(19) Europäisches Patentamt
European Patent Office
Office européen des brevets

(11) **EP 1 800 633 A1**

(12) **EUROPEAN PATENT APPLICATION**

(43) Date of publication:
**27.06.2007 Bulletin 2007/26**

(51) Int Cl.:
*A61F 6/22* (2006.01)       *A61B 17/12* (2006.01)

(21) Application number: **05257920.8**

(22) Date of filing: **20.12.2005**

(84) Designated Contracting States:
**AT BE BG CH CY CZ DE DK EE ES FI FR GB GR
HU IE IS IT LI LT LU LV MC NL PL PT RO SE SI
SK TR**
Designated Extension States:
**AL BA HR MK YU**

(71) Applicant: **FEMCARE LIMITED
Nottingham NG8 6AR (GB)**

(72) Inventors:
• **Johnson, David
Nottingham NG8 6AR (GB)**

• **Filshie, Marcus
Nottingham NG8 6AR (GB)**
• **Magos, Adam Laszlo
Herfordshire EN5 4EE (GB)**

(74) Representative: **Baker, Colin John
Eric Potter Clarkson LLP
Park View House
58 The Ropewalk
Nottingham NG1 5DD (GB)**

Remarks:
Amended claims in accordance with Rule 86 (2) EPC.

(54) **Sterilisation device**

(57)    The invention relates to a sterilisation device comprising a plug (50) which is capable of occluding a fallopian tube, the plug having an external thread (55) thereon wherein the thread has a root length in the range 0.8 to 1.5 mm and a pitch in the range 1.55 to 2.0 mm so that, upon rotational insertion of the plug into a fallopian tube, the plug grips the walls of the fallopian tube and resists removal from the fallopian tube. The device may form part of a sterilisation assembly further including a driver (105) configured to co-operate with the head (65) of the plug facilitating the transfer of torque from the driver to the sterilisation device. Also described is a method of female sterilisation comprising the steps of introducing the device into a uterine cavity and screwing the device into a fallopian tube. This may be executed using the assembly of the invention, or other methods.

figure 3

EP 1 800 633 A1

**Description**

The invention relates to a sterilisation device for use in occluding a fallopian tube of a female. In particular, the invention relates to a sterilisation device which resists removal from the fallopian tube. Further aspects of the invention relate to an assembly for positioning the device in the fallopian tube, and to a method of female sterilisation.

Various methods of female sterilisation are currently in use, these mostly involve division, excision or external occlusion of the fallopian tubes and laparotomy or laparoscopy.

Hysteroscopic/vaginal methods which avoid abdominal surgery have also been tried. These include the use of pre-formed plugs of various designs which can be inserted into the tubal ostium without any fixation, or are fixed in place by spines or a perforating claw, or because they swell after insertion (or a combination of these properties). A technique based on the injection of a formed in situ silicone plug is also known. The tubal ostia can also be coagulated using thermal energy using a thermal coagulator, electrodiathermy or laser, blindly or under hysteroscopic vision. Finally, sclerosing chemicals can be injected into the fallopian tune or uterine cavity, again blindly or under hysteroscopic control.

However, sterilisation at laparotomy involves major surgical trauma, leaves a relatively large external scar, and requires a general anaesthetic. Laparoscopic sterilisation can be performed under local anaesthesia (although generally it is not), but still risks all the complications of laparoscopy, principally intra-abdominal trauma related to the blind insertion of needles and cannulae. Neither approach to sterilisation is suitable for out-patient treatment.

Hysteroscopic/vaginal methods of tubal occlusion are suitable for out-patient treatment. However, techniques utilising pre-formed plugs were abandoned because of a high failure rate resulting from dislodgement. The formed in situ silicone plugs involve multiple applications in over 10% of patients to achieve bilateral insertion, and the inconvenience of a follow-up hysterosalpinogram X-ray to check for tubal occlusion. The same investigation is required for methods involving thermal coagulation of the proximal fallopian tube, with the additional disadvantages of a high complication rate of the procedure itself and a high pregnancy rate; as a result these techniques have largely been abandoned. The injection of irritant sclerosing agents has met with variable success, but once more several treatments may be required, a post-sterilisation hysterosalpinogram is necessary, and even if this shows bilateral tubal occlusion, tubal recanalisation can still occur.

Sterilisation plugs comprising helical threads are also known, for instance from WO 97/12569 (Magos). This document describes a threaded plug which may be screwed into the fallopian tube, however, this plug is prone to ejection from the fallopian tube leading to an unacceptable failure rate in sterilisations using this product.

Accordingly, there remains a need in the art for simple helically threaded plugs which are reliably retained within the fallopian tube after insertion. The subject invention seeks to address this problem through optimisation of the features of the plug.

According to a first aspect of the invention there is provided a sterilisation device comprising a plug which is capable of occluding a fallopian tube the plug having an external thread thereon wherein the thread has a large root length and a large pitch. Upon rotational insertion of the plug into a fallopian tube, the plug grips the walls of the fallopian tube and resists removal from the fallopian tube.

Unlike laparotomy or laparoscopy the inventive sterilisation device is suitable for use out-patient treatment without general anaesthesia, leaves no external scars and minimises the risk of intra-abdominal trauma. Further, although removable if required (for instance is intended for contraceptive use only), the device is unlikely to dislodge or migrate as a result of contractions in the fallopian tube as it attempts to expel the foreign body. Tests have shown that the inventive device is more difficult to dislodge than other types of screw thread. This would result in markedly reduced failure rates when compared to earlier threaded plugs.

In addition, the inventive plug allows occlusion to be achieved in one application with immediate sterilising effect. This was not possible with earlier plugs which were formed in situ. Further, there is no need for a follow-up hysterosalpinogram as is required where in situ plug formation is used. Additionally, there is no thermal damage to the surrounding tissue and tubal recanalisation cannot occur.

Typically, the root length will be in the range 0.8 - 1.5 mm and the pitch independently in the range 1.55 - 2.0 mm. The relatively large pitch and large root length offers a device with a large shear area reducing the likelihood of shear failure in the threads formed from the bodily tissue.

In one embodiment, the thread form is symmetrical; preferably, where the thread form is symmetrical the thread angle is in the range 10 - 30° from a minor axis of the device. More preferably, the angle is in the range 15 - 25°, most preferably 17 - 21°. The narrow thread angle increases the axial force exerted by the screw improving the resistance of the device to removal from the fallopian tube. In particular, resistance to ejection of the plug by the body is improved.

In an alternative embodiment, the thread form is asymmetrical wherein the asymmetrical thread form comprises a leading flank and a trailing flank and wherein the angle between the trailing flank and a minor axis of the plug is smaller than the angle between the leading flank and a minor axis of the plug. This configuration, with a steeper flank on the side of the thread placed under pressure during ejection or pull-out of the device, offers improved resistance to removal of the plug because it is more difficult pull the plug past the tissue which has flowed into the area behind the thread as this tissue will not easily slide past a steeper flank. Accordingly, the device is better retained an position.

It is preferred that, in instances where the thread form is asymmetric, the leading flank has a thread angle in the range 20 - 45° from a minor axis of the plug. More preferably, this angle is 30 - 45°, most preferably 40 - 45°. Typically, the trailing flank has a thread angle in the range 0 - 25° from a minor axis of the plug, more preferably, 0-15°, most preferably 0 - 5°.

Typically the device will be elongate, although it may be of any shape which would occlude the fallopian tube. It is preferred that the plug is tapered with the widest point distal to the tip of the plug. Where this is the case the thread may be cylindrical or it may be tapered either to the same angle as the plug, or to a different angle. It is preferred that the plug be tapered because this has been found to increase the pull-out force required to remove the device from the fallopian tube. Preferably the degree of taper is in the range 1- 5°, more preferably 2 - 4°.

In preferred embodiments the major diameter of the device will be in the range 4.0 - 1.8 mm at the end distal to the tip of the plug, tapering to 3.0 - 1.5 mm at the end proximal to the tip. It is preferred that the major diameter of the device will not exceed 4.0 mm. Typically, but not always, the minor diameter of the device will be in the range 2.8 - 0.6 mm at the end distal to the tip of the plug, tapering to 0.6-0.3 mm at the end proximal to the tip.

Typically, the device will have a length in the range 1.0.0 - 20.0 mm, preferably 12.0 -18.0 mm. Of the total length of the plug, a head of the plug (i.e. the shaped part of the plug beyond the threaded portion and distal to the tip of the device) would typically have a length in the range 3.0 - 6.0 mm and the thread, independently, have a length in the range 7.0 - 14.0 mm. In this instance, the length of the thread corresponds to the distance between the tip of the device and the end of the thread distal to the tip and is more preferably 9.0 - 12.0 mm. The thread may be contiguous with the head or there may be a gap on the plug between the head and the thread.

The thread height of the device will be large, often in the range 0.6 - 1.2 mm. A large thread height increases the shear area reducing the likelihood of shear failure in the threads formed from the bodily tissue. It is preferred that the thread height remain constant along the length of the plug, however, it is also envisaged that this may increase or decrease towards the tip of the device.

It is preferred that the tip of the plug is flattened, however, it may be configured in any other way known in the art, for instance it may be pointed, rounded, cupped, die pointed or rolled. A flattened tip offers the advantage that the force applied by the fallopian tube when it contracts to expel the plug (as a foreign body) is at around 45° to the surface of the tip. As a result, approximately half of the force is deflected parallel to the plane of the flattened tip reducing the force applied in an expulsive direction.

It is more preferred that the flattened tip has rounded edges as these facilitate location of the device into the tube and reduce the likelihood of the tip of the plug ripping or tearing the body tissue. In the most preferred embodiments the degree of rounding is in the range R0.1 - R0.4.

The device may also optionally include additional anti-ejection means. These may be in the form of one or more incisions in the thread which (once the device is positioned) allow the soft tissue of the fallopian tube to occupy the space left by the incision. This embedding of the tissue in the incision helps to prevent rotation of the plug once positioned, making it more difficult for the device to unscrew and hence be removed. The shape of the incisions is not critical and they may be either angular or curved, typically they will be angular for ease of manufacture and to provide more resistance to movement once the body tissue is embedded in the incision. In one suitable configuration the incision will be in the shape of a 'V' shaped notch cut out of the thread. Where the incision is 'V' shaped, it is preferred that the angle between the sides of the 'V' is approximately 45°.

In such embodiments, it is preferred that there are two incisions one each in the first two rotations of the thread at the end distal to the tip of the plug. It is more preferred that the incision in the first rotation is aligned with the incision in the second rotation. Most preferably there are four incisions, two each in the two rotations of the thread distal to the tip of the plug, wherein the incisions are equally spaced on diametrically opposing edges of the thread so that the incisions in the first rotation of the thread are aligned with the incisions in the second rotation of the thread.

The notches may have a depth corresponding to the entire thread height, or may have a depth which is only a portion of the thread height. It is not necessary for the depth of each notch to be the same, although preferably they will be of substantially equal depth. In preferred embodiments, the notch will have a depth in the range 20 - 80% of the thread height, more preferably, 30 - 70%, most preferably 45 - 70% of the thread height.

Preferably, the plug has a head on the opposite end to the tip which head has at least one formation allowing torque to be applied to the plug by a driver. The device may be applied to the fallopian tube using any known application device. Preferably, however a conventional driver will be used.

In preferred embodiments the head is polygonal, more preferably the head has a cross-section through the minor axis which is a regular polygon such as an octagon or hexagon. Most preferably the head is hexagonal. Typically the diameter of the head will be in the range 1.0 - 4.0 mm.

Accordingly, the driver may include a socket which is typically configured to co-operate with the polygonal head of the plug so as to receive and engage the head so that torque applied to the driver is transferred to the plug. The driver is preferably elongate.

Therefore, according to a further aspect of the invention there is provided a sterilisation assembly comprising a sterilisation device and a driver configured to co-operate with the head of the plug facilitating the transfer of torque from the driver to the sterilisation device.

Other co-operating shapes of head and driver socket may be used, for instance slots in one and ridges or protrusions in the other. In one aspect, the driver may comprise a polygonal protrusion which mates with a co-operating socket in the head of the plug. The head may have an annular groove and the socket a circumferential ridge (or vice versa) which co-operate to connect socket and head together. Alternatively or additionally, the socket may comprise a clamp (e.g. it may have arms which are radially expansible or contractible) so as to receive the head in a tight fit. Other connecting and locking means may be provided to lock the device to the driver.

The plug and driver may be at least partially enclosed within an outer sleeve for ease of manipulation of the plug and driver in the uterus. The outer sleeve and driver can both be laterally flexible but preferably both are fairly resistant to longitudinal compression and tension. The driver and plug can preferably move axially within the outer sleeve, and movement .(e.g. axial movement) of the plug and driver relative to the outer sleeve can be a useful means of attaching and releasing the plug from the driver in the uterus.

Means may be provided in the outer sleeve to include lateral bending of the outer sleeve (and of the driver therein) in response to manipulation of controls at the end of the sleeve remote from the plug. This allows manipulation of the sleeve, driver and plug in the uterus. Alternatively, or additionally, the sleeve, driver and plug can be passed through a channel in an operating hysteroscope or other such device.

The head may be provided with means to facilitate the removal of the plug. These may comprise formations to assist the gripping of the head with forceps, or by the driver. The means may also be an aperture passing through the head of the plug which may engage with a hook or wire. Once connected to the hook or wire, rotation of the plug could be effected, unscrewing the device and hence facilitating non-traumatic removal of the device from the fallopian tube. Where present, the aperture will typically be cylindrical for ease of manufacture and will pass entirely through the head. The diameter of the aperture will preferably be in the range 0.5 -1.5 mm, and the aperture will usually be positioned towards the end of the head most distal to the tip of the device. For instance, the aperture may be placed somewhere in the range 1.0 - 3.0 mm from this end of the head.

The device may be made from a variety of bio-compatible materials, however preferably it will comprise a rigid inert plastics material. Often the device will be radiopaque or visible to X-rays or ultrasound scanning.

According to a further aspect of the invention there is provided a method of female sterilisation comprising the steps of introducing a device as described in the first aspect of the invention into a uterine cavity and screwing the device into a fallopian tube. Preferably, the device is located within the driver prior to introduction into the uterine cavity, to facilitate manipulation of the device external to the patient. Where the driver is used, the device must be released from the driver once it has been screwed into the fallopian tube. Most preferably, the device is located in an end of the driver and retained therein using a sleeve. The sleeve may be moved relative to the driver so that retention of the device is achieved by sliding the sleeve to the end of the driver so that pressure is applied to the device located within the driver. Release from the driver may be achieved by withdrawal of the sleeve from the end of the driver.

It will be understood that, as the device is intended to be removable where necessary, the term 'sterilisation' is intended to encompass not only permanent sterilisation of the female, but also contraceptive use of the device where it is intended that the device be removed after a period of time.

It will be clear to the skilled reader that, unless otherwise stated, all parameters appearing in this application are to be taken as modified by the word 'about'. Additionally, each feature described in the application may be taken in combination with any other feature described in the application with the exception that asymmetric and symmetric thread forms may not be combined.

An embodiment of the invention will now be described in detail by way of nonlimiting example only with reference to the accompanying drawings in which:-

Figure 1 is a schematic representation of a cross-section through a typical screw thread;
Figure 2 is a side view of a device according to the invention;
Figure 3 is a side view of a device according to an alternative embodiment of the invention;
Figure 4 is a perspective view of a device according to a further embodiment of the invention;

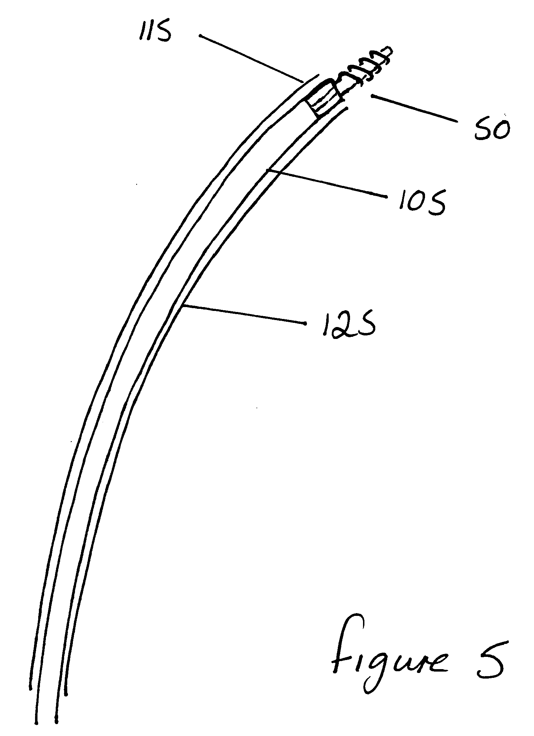

Figure 5 is a side view of a sterilisation assembly according to the invention;

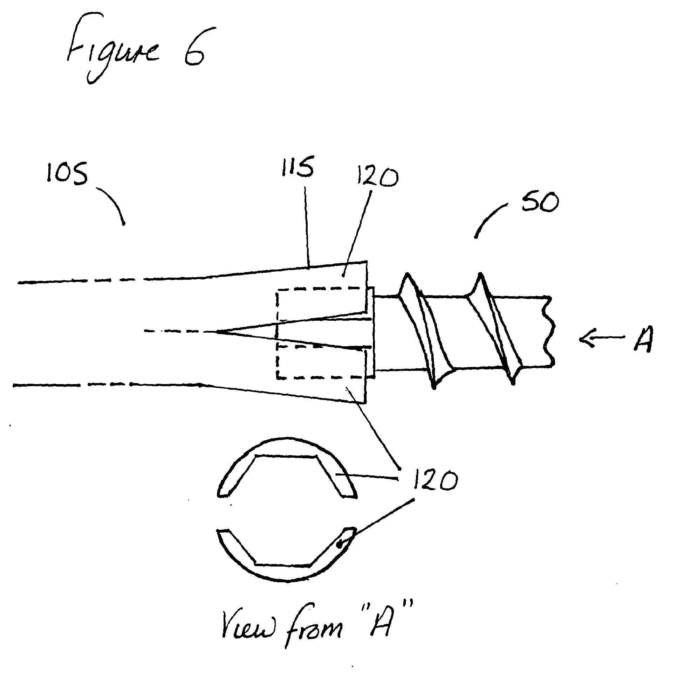

Figure 6 is a diagrammatic side view of part of the assembly of Figure 5;
Figure 7 is a flow diagram of test set up;
Figure 8 is a graph illustrating the pull-out force required for a range of devices including those of the embodiments of Figures 2 and 3; and
Figure 9 is a graph illustrating the relative performance ratios of the devices described in Figure 7.

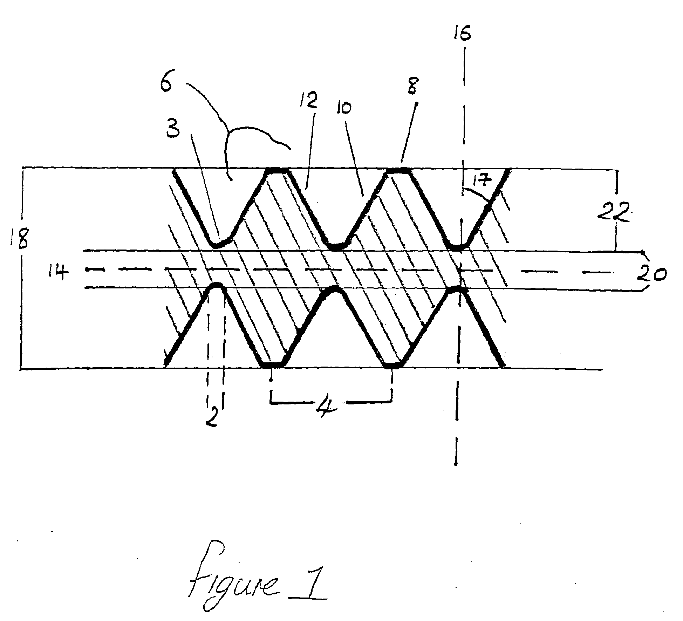

The 'root length' 2, illustrated in Figure 1, is the distance between the base of two flanking surfaces of a thread whether internal or external wherein the 'root' 3 is the bottom of a groove between the two flanking surfaces of the thread.

The 'pitch' 4 of the thread of a sterilisation device is the distance, measured parallel to the major axis 14 of the device, between corresponding points on adjacent surfaces in the same axial plane.

The 'thread form' 6 of the thread is the shape of the thread and may be symmetrical, or asymmetrical.

The 'crest' 8 of the thread is the prominent part of the thread.

The 'flanks' 10, 12 of the thread are the straight sides that connect the crest 8 and the root 3. A 'leading flank' 10 of the thread is the flank of the thread found nearest to a tip of the device. A 'trailing flank' 12 of the thread is the flank found nearest to a head of the device. Typically, the leading flank 10 will have an external angle, however, the angle of the trailing flank 12 may be external or internal.

The 'major axis' 14 of the device is the rotational axis running through the centre of a plug and along the length of the plug. A 'minor axis' 16 of the device is any axis perpendicular to the major axis 14 which cuts through the device parallel to the direction of extension of the thread from the root 3.

The 'thread angle' 17 is the angle formed between the flank of the thread and a minor axis 16 of the device where the minor axis 16 cuts through the plug at the base of the flank, and the thread angle 17 is determined in clockwise rotation from this axis.

The 'major diameter' 18 of a thread is the diameter of the imaginary co-axial cylinder that just touches the crest 8 of an external thread or the root of an internal thread. The 'minor diameter' 20 is the diameter of the cylinder that just touches the root 3 of an internal thread.

The 'thread height' 22 is the distance along a minor axis 16 between the root 3 and crest 8 of the thread; or in other words, half the difference between the major diameter 18 and the minor diameter 16 at that point along the device.

Accordingly, the pitch 4 can be defined as:

$$Pitch = root + thread\ height\ (cosA + cosB) + tip\ length$$

Where A is the leading flank 10 angle and B is the trailing flank 12 angle. If the distance between the tip and the end of the thread proximal to the tip is not considered, the pitch 4 can be defined as:

$$Pitch = root + thread\ height\ (cosA + cosB)$$

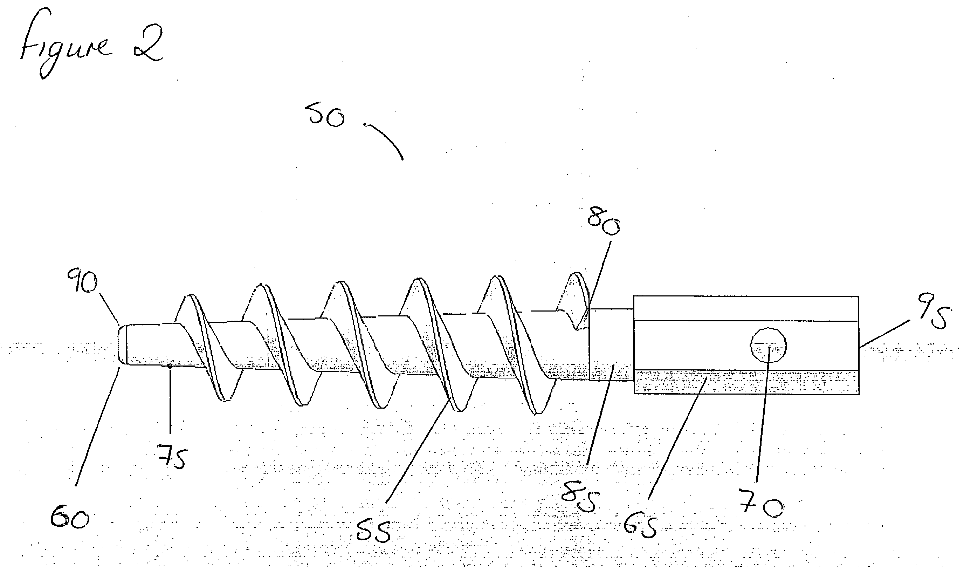

According to one embodiment of the invention there is provided a sterilisation device 50 comprising an elongate plug 50 having at a first end a flattened tip 60, at a second end a head 65 and an external self-tapping thread 55 therebetween (Figure 2). The device 50 of this example may be formed from implantable grade polyethylene which in this embodiment is radiopaque.

The root length 2 of the device 50 described in this embodiment is 1.1 ± 0.1 mm and the pitch 4 is 1.75 ± 0.1 mm. The thread 55 in this embodiment is symmetrical and has a thread angle 17 of 19° from minor axis 16 of the device 50. In this example the plug 50 has a 3° taper with the widest point at the head 65 of the device 50 and the taper towards the tip 60. The thread 55 of this example also tapers so that the thread height 22 remains constant at 0.8 ± 0.1 mm along the length of the plug 50.

The plug 50 of Figure 2 is sized so as to fit within the fallopian tubes of a human female, and may be adapted as necessary for the size of the female whether this is as a result of the natural difference in sizes between human females, or as a result of use of the device 50 to sterilise a female of another species. The major diameter 18 of the plug 50 of this embodiment is 3.2 ± 0.05 mm at the end of the thread distal to the tip of the device 80 tapering to 2.08 ± 0.05 mm at the end of the plug proximal to the tip 75. The minor diameter 20 is therefore 1.6 ± 0.05 mm at the end of the thread distal to the tip of the device 80 tapering to 0.48 ± 0.05 mm at the end of the plug proximal to the tip 75. The overall length of the device 50 of this example is 16.5 ± 0.1 mm of which the head comprises 5 ± 0.4 mm and a thread length (i.e. the distance between the tip 60 of the device and the end 80 of the thread 55 nearest to the head 65) of 10.5 ± 0.1 mm. In this example there is a gap 85 between the end of the thread 55 and the head 65 of approximately 1.0 mm, although this is not essential.

The tip 60 of the plug 50 is flattened, with the edges 90 rounded to prevent damage to the body tissue. The degree of rounding for this embodiment is R0.2.

The head 65 of this embodiment is hexagonal and of distance between each pair of opposing sides 2.1 ± 0.05 mm. A cylindrical aperture 70 of 1.0 ± 0.4 mm diameter passes through the head 65, between the centre of two opposing sides of the hexagon. The centre of the aperture 65 is positioned, in this example, 2.0 ± 0.4 mm from the end of the device at which the head is found 95. The aperture 70 allows a handle or wire (not shown) to be passed therethrough.

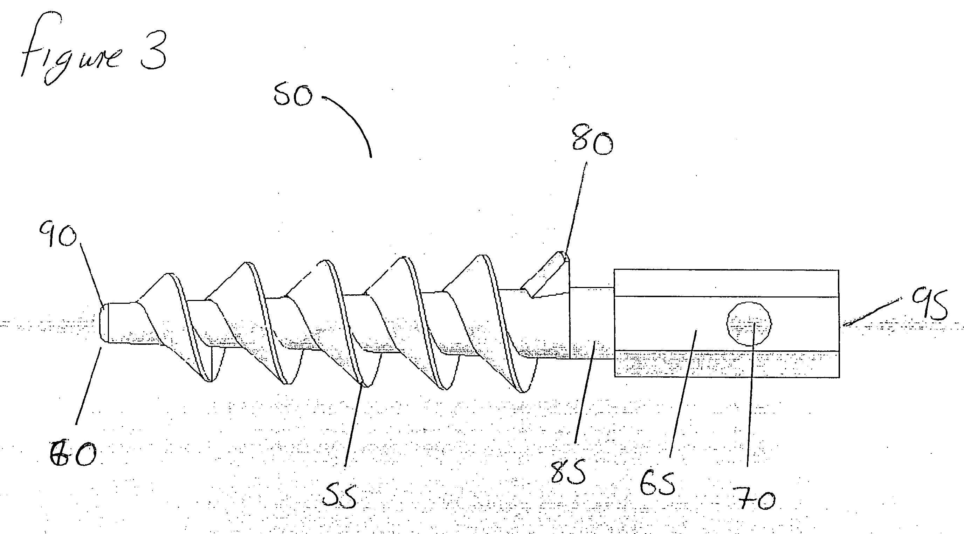

An alternative embodiment of the invention, shown in Figure 3, has the same overall dimensions as the embodiment of Figure 2 with the following exceptions. The device 50 of this embodiment has an asymmetric thread form 6, having a thread angle of 45 ± 0.3° for the leading flank 10 and 2 ± 0.3° for the trailing flank 12. This difference in thread configuration results in a smaller root length 2 of 0.82 ± 0.05 mm. The asymmetric thread form 6 resists removal of the plug 50 by trapping the body tissue behind and underneath the slight overhang formed by the trailing flank 12.

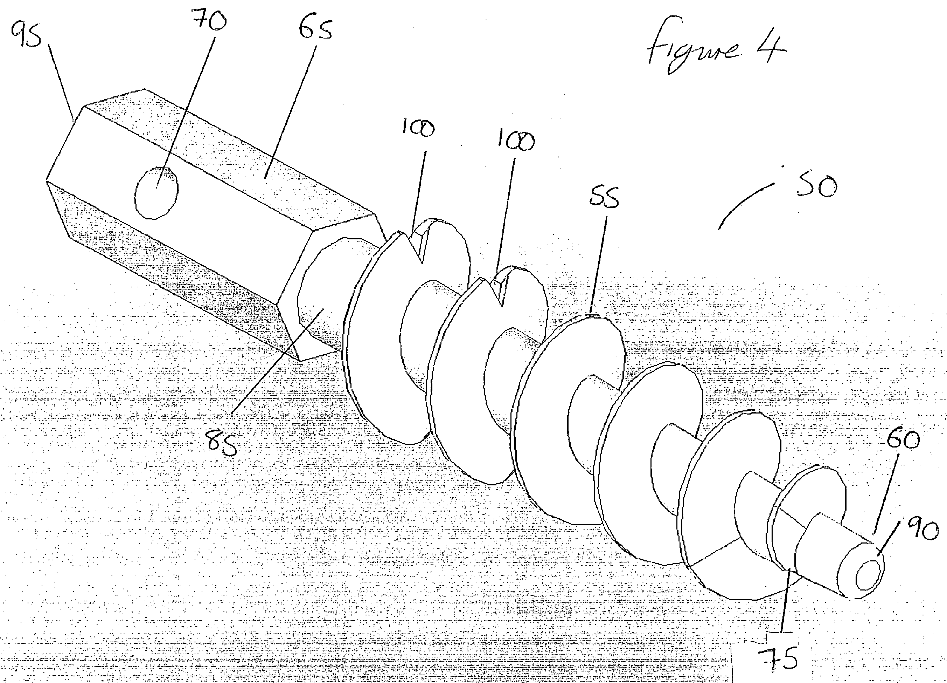

A third embodiment of the invention is also described in which the device 50 of Figure 2 has been modified by the inclusion of four incisions 100 in the thread 55. These V-shaped incisions 100 are positioned two each on the first two rotations of the thread 55 nearest the head 65 of the plug 50. The incisions 100 are equally spaced on diametrically opposing edges of the thread 55 so that the incisions 100 in the first rotation of the thread 55 are aligned with the incisions 100 in the second rotation of the thread 55 (as shown in Figure 4). The depth of the incisions of this example are approximately 55% of the thread height (i.e. roughly 0.4 mm ± 0.1 mm from the base of the 'V').

The head 65 of each of the devices 50 of Figures 2 to 4 (and thus the thread 55) may be rotated by a driver 105 which comprises a hollow tube with an inner hexagonally profiled surface to engage with the hexagonal head 65 of the device 50. In this embodiment the distal end 115 of the driver 105 is slotted to allow divergence of the end 115 around the head 65 of the device 50 so that the device 50 is securely held within the divergent arms of the end 115. The driver 105 is formed of flexible plastics material and can bend laterally but is preferably resistant to torsional, compressive and tensioning forces. In this example, the driver 105 measures approximately 25 cm by 2.8 mm by 2.8 mm and is designed for a human female, although measurements may be altered for a non-human subject.

In this example a sleeve 125 in the form of an outer sheath is also provided, comprising a hollow tube with thin walls resistant to torsional compressive and tensioning forces, but able to bend laterally. The sleeve 125 is dimensioned to fit over the driver 105 and has, in this instance has approximate dimensions of 20 cm by 3.0 mm by 3.0 mm. When the sleeve 125 is slid over the arms 120 of the driver 105, the sleeve 125 can compress the arms 120 against the head 65 of the device 50, thereby locking the device 50 in position in the driver 105. Sliding movement of the sleeve 125 away from the end 115 allows divergence of the arms 120 and release of the device 50.

All components are preferably capable of being sterilised using conventional techniques, including but not limited to gamma radiation or autoclaving at 121°C for 20 minutes.

In use, the device 50 may be located in the end 115 of the driver 105 and the sleeve 120 slid therealong so as to close the arms 120 at the end around the head 65 thereby holding the device 50 in place. The assembly is then introduced into the uterine cavity, optionally using a hysteroscope fitted with a suitable operating channel. Under hysteroscopic vision, the tip 60 of the device 50 is directed towards the tubal opening of the fallopian tubes, and the device 50 is screwed into the proximal fallopian tube by clockwise rotation of the driver 105, which can be manipulated externally of the patient. Once in place, the device 50 can be released' from the driver 105 by withdrawing the sleeve 125 thereby allowing the arms 120 at the end 115 to diverge and releasing the device 50 from the driver 105. The sleeve 125 and driver 105 are thereafter removed from the uterus and the procedure can be repeated on the contralateral side.

For removal of the device 50 from the fallopian tubes, the end 115 of the driver 105 can be located on the head 65 of the device 50 and the sleeve 125 slid therealong to clamp the end 115 around the head 65. The device 50 can thereafter be unscrewed anti-clockwise from the fallopian tube and removed from the uterine cavity.

In the event that the device 50 cannot be unscrewed using the driver 105, hysteroscopic forceps can be used to pull the handle or wire (not shown) at the end of the device 50 to dislodge it from the fallopian tube.

<u>Examples</u>

The examples below describe the testing of eight different devices including the devices of Figures 2 and 3 (devices X2 and X1 respectively). Each plug was tested 20 times in polystyrene and in potato.

The prototype plugs were manufactured in a rigid plastics material.

The plugs tested in addition to X2 and X1 of Figures 2 and 3 are outlined in Table 1 below.

Table 1

| Image | Title | Pitch | Flank Angle |
|---|---|---|---|
| | Test 1-lp | 1.50mm | 50° |
| | Test 2-35 | 0.90mm | 35° |
| | Test 2-60 | 0.90mm | 60° |
| | Square Thread | 1.20mm | N/A |
| | CT | 0.90mm | 50° |
| | CC | 0.90mm | 50° |

## TEST METHOD

**Sample Selection:** The tests were completed using two different samples, a synthetic sample, in this case medium density polystyrene, and potato.

**Sample Size:** each plug was tested 20 times using medium density polystyrene and potato. All the samples were selected at random. It was assumed that the variability in the polystyrene/potato texture would produce an even distribution of errors.

**Equipment Used:**

- Test Screw
- Screw Applicator
- Polystyrene Sample/Potato Sample
- Test Mount
- Test Rig
- Salter Super Samson Spring Gauge
- Spike (paper clip)
- Fishing Line

**Loading the Specimens:** a pilot hole was introduced into the specimen using a spike, a 1.5 mm drill bit was then used to drill a hole down the middle of the specimen. Once this had been done, the end without a hole was inserted into the front entrance on the front of the test mount and pushed down the canal until the end cleared the back of the test mount. The top clamp was then applied to secure the specimen in place.

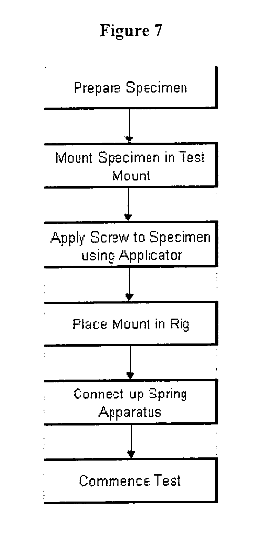

The plug was then threaded with a short length of fishing line through the hole in the rear shaft. This was tied in a loop. The plug was then loaded into the screw applicator and screwed into the loaded specimen. Care was taken to ensure that the screw was kept straight and level as to not disrupt the thread. The plug was inserted until the thread was no longer visible. The test mount was then loaded into its cradle in the test rig and the fixed end of the spring gauge hooked through the loop in the screw. The other end in the loop was attached to the ratchet which was then wound gently to take up the slack in the fishing line to complete test set up. An outline of the test set up is provided in Figure 7.

**Completing the Test:** the ratchet was twisted one click at a time in a clockwise direction, noting the load increments on the spring gauge after each twist Care was taken to ensure that a slow and constant pace was used throughout the test. When the screw was pulled out from the specimen the last read value from the spring gauge in the table was noted. This procedure was repeated 20 times for each of the plugs to be tested.

**Data Conversion:** once the tests were completed, the data was processed to convert the load into a force ($0.01 X(\text{grams}) = Y(\text{Newtons})$). Test anomalies were then assessed and anomalous results omitted from the test data. Averages of the remaining results were then produced.

**ANALYSIS**

Two different sized holes were used in the polystyrene tests. Initially, a 1.5 mm diameter hole was used, however, it was found that X2 and X1, both with tip diameters of less than 1.0 mm required a smaller hole in order to grip. Accordingly, after 10 set of results had been collected, the diameter was reduced from 1.5 mm to 1.0 mm. Research had shown that the diameter of the uteral tubal junction, where the plug is designed to be placed, is potentially only 0.1 mm (Nilsson & Reinius, 1969) but more recently the diameter at the entrance is reported to be 0.5 - 1.5mm (Merchant et al, 1983).

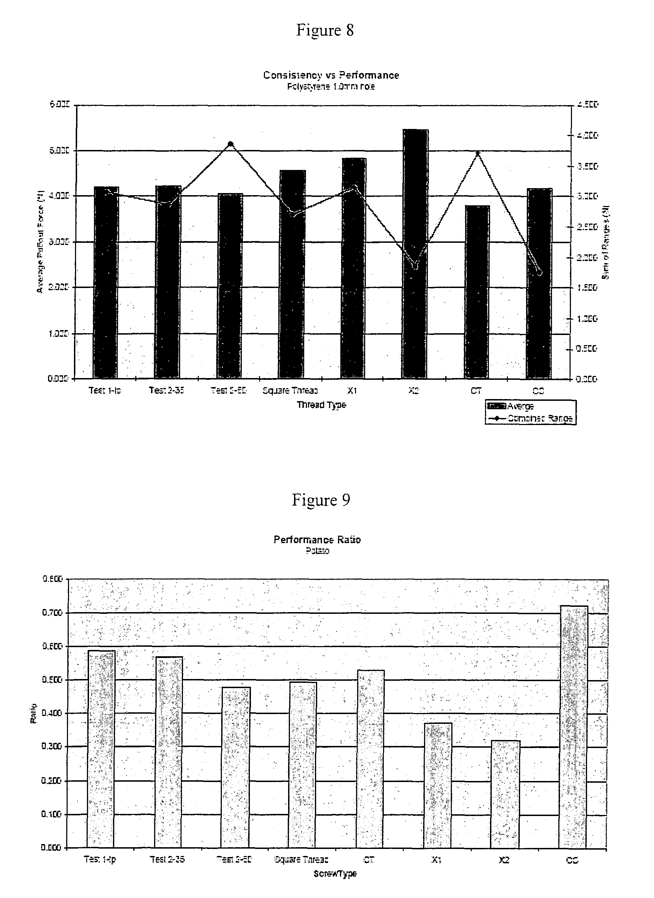

Plug 2-35, came 4th for both the performance and consistency in the 1.0 mm polystyrene hole (see Figure 8) but 7th and 8th in the 1.5mm hole. Each of plugs 2-60 and CT, had one anomalous result. Both anomalies occurred in the 1.5 mm test suggesting that the 1.0 mm data is more reliable. Accordingly, on the basis of the 1.0 mm test data plug 2-35 came middle of the field and the other two, 2-60 and CT, respectively last.

Plug 2-35 out performed 2-60 in terms of pullout strength, this is a basic comparison test as all other features on these two plugs were identical, indicating that a steeper flank angle is more desirable to increase the pullout strength.

Plugs X1, X2 and square thread performed the best in terms of pullout strength, all have the steepest trailing flank angle. Plugs X1 and X2 both have the greatest thread depth and both outperformed all the other plugs also suggesting that a greater thread depth is needed to increase pullout strength (Figures 8 and 9).

Test 1-1p outperformed both plugs 2-35 and 2-60 in the majority of tests. Plug 1-1p has a larger pitch than both 2-35 and 2-60 which have equal pitch and a flank angle that lies between that of 2-35 (35°) and 2-60 (60°) suggesting that a larger pitch is required to further improve the pullout strength.

The analysis consisted of two main parts, performance (Figure 9) and consistency (Figure 8). The consistency was calculated by combining the range with the interquartile range. This was to analyse the spread of the results as a smaller spread is more desirable. The performance was based on the average pull out strength of the plug. These two sets of data were combined to produce a performance ratio, the smaller the ratio the better the overall performance.

It is clear from the results above and illustrated in the graphs of Figures 8 and 9 that the inventive embodiments (X2 and X1) performed significantly better both in potato and polystyrene than the other plugs tested.

**Claims**

1. A sterilisation device comprising a plug which is capable of occluding a fallopian tube, the plug having an external thread thereon wherein the thread has a root length in the range 0.8 to 1.5 mm and a pitch in the range 1.55 to 2.0 mm so that, upon rotational insertion of the plug into a fallopian tube, the plug grips the walls of the fallopian tube and resists removal from the fallopian tube.

2. A sterilisation device according to claim 1 wherein the thread has a thread form which is symmetrical.

3. A sterilisation device according to claim 2 wherein the symmetrical thread form has a thread angle in the range 10 to 30° from the minor axis of the plug.

**4.** A sterilisation device according to claim 1 wherein the thread has a thread form which is asymmetrical.

**5.** A sterilisation device according to claim 4 wherein the asymmetrical thread form comprises a leading flank and a trailing flank and wherein the angle between the trailing flank and a minor axis of the plug is smaller than the angle between the leading flank and a minor axis of the plug.

**6.** A sterilisation device according to claim 5 wherein the leading flank has a thread angle in the range 20 to 45° from the minor axis of the plug.

**7.** A sterilisation device according to claim 4 or claim 5 wherein the trailing flank has a thread angle in the range 0 to 25° from the minor axis of the plug.

**8.** A sterilisation device according to any preceding claim wherein the plug is elongate.

**9.** A sterilisation device according to any preceding claim wherein the plug is tapered.

**10.** A sterilisation device according to any preceding claim of total length in the range 10-0 to 20.0 mm.

**11.** A sterilisation device according to any preceding claim wherein the length of a head of the plug is in the range 3.0 to 6.0 mm.

**12.** A sterilisation device according to any preceding claim wherein the length from a tip of the plug to the end of the thread distal to the tip is in the range 7.0 to 14.0 mm.

**13.** A sterilisation device according to any preceding claim wherein the thread height is in the range 0.6 to 1.2 mm.

**14.** A sterilisation device according to any preceding claim wherein a major axis of the plug has a maximum dimension not exceeding 4.0 mm.

**15.** A sterilisation device according to any preceding claim wherein the plug comprises a flattened tip.

**16.** A sterilisation device according to claim 15 wherein the tip has rounded edges.

**17.** A sterilisation device according to any preceding claim comprising anti-ejection means.

**18.** A sterilisation device according to claim 17 wherein the anti-ejection means comprises at least one incision in the thread.

**19.** A sterilisation device according to claim 18 having four incisions two each in the two rotations of the thread nearest to a head of the plug.

**20.** A sterilisation device according to claim 19 wherein the incisions are equally spaced on diametrically opposing edges of the thread so that the incisions in the first rotation of the thread are aligned with the incisions in the second rotation of the thread.

**21.** A sterilisation device according to any preceding claim wherein the plug comprises a head adapted to allow torque to be applied to the plug by a driver.

**22.** A sterilisation device according to claim 21 wherein the head is polygonal.

**23.** A sterilisation device according to any preceding claim additionally comprising means for removal of the plug.

**24.** A sterilisation device according to claim 23 wherein the means for removal of the plug comprises an aperture passing through the head of the plug.

**25.** A sterilisation assembly comprising a sterilisation device according to any preceding claim and a driver configured to co-operate with the head of the plug facilitating the transfer of torque from the driver to the sterilisation device.

**26.** A sterilisation assembly according to claim 25 further comprising a sleeve.

**27.** A method of female sterilisation comprising the steps of:

introducing a device according to any of claims 1 to 24 into a uterine cavity; and
screwing the device into a fallopian tube.

**28.** A method according to claim 27 comprising the additional step of locating the device in a driver prior to introduction into the uterine cavity.

**29.** A method according to claim 28 comprising the additional step of releasing the device from the driver after the device has been screwed into the fallopian tube.

**30.** A method according to claim 28 or claim 29 wherein the device is secured in the driver using a sleeve.

**Amended claims in accordance with Rule 86(2) EPC.**

**1.** A sterilisation device (50) comprising a plug (50) which is capable of occluding a fallopian tube, the plug having an external thread (55) thereon wherein the thread has a root length (2) in the range 0.8 to 1.5 mm and a pitch (4) in the range 1.55 to 2.0 mm so that, upon rotational insertion of the plug into a fallopian tube, the plug grips the walls of the fallopian tube and resists removal from the fallopian tube.

**2.** A sterilisation device according to claim 1 wherein the thread (55) has a thread form (6) which is symmetrical.

**3.** A sterilisation device according to claim 2 wherein the symmetrical thread form (6) has a thread angle (17) in the range 10 to 30° from the minor axis (16) of the plug (50).

**4.** A sterilisation device according to claim 1 wherein the thread (55) has a thread form (6) which is asymmetrical.

**5.** A sterilisation device according to claim 4 wherein the asymmetrical thread form (6) comprises a leading flank (10) and a trailing flank (12) and wherein the angle between the trailing flank and a minor axis (16) of the plug (50) is smaller than the angle between the leading flank and a minor axis of the plug.

**6.** A sterilisation device according to claim 5 wherein the leading flank (10) has a thread angle (17) in the range 20 to 45° from the minor axis (16) of the plug (50).

**7.** A sterilisation device according to claim 4 or claim 5 wherein the trailing flank (12) has a thread angle (17) in the range 0 to 25° from the minor axis (16) of the plug (50)

**8.** A sterilisation device according to any preceding claim wherein the plug (50) is elongate.

**9.** A sterilisation device according to any preceding claim wherein the plug (50) is tapered.

**10.** A sterilisation device according to any preceding claim of total length in the range 10.0 to 20.0 mm.

**11.** A sterilisation device according to any preceding claim wherein the length of a head (65) of the plug (50) is in the range 3.0 to 6.0 mm.

**12.** A sterilisation device according to any preceding claim wherein the length from a tip (60) of the plug (50) to the end (80) of the thread (55) which is distal to the tip (60) is in the range 7.0 to 14.0 mm.

**13.** A sterilisation device according to any preceding claim wherein the thread height (22) is in the range 0.6 to 1.2 mm.

**14.** A sterilisation device according to any preceding claim wherein a major diamenter (14) of the plug (50) does not not exceed 4.0 mm.

**15.** A sterilisation device according to any preceding claim wherein the plug (50) comprises a flattened tip (60).

**16.** A sterilisation device according to claim 15 wherein the tip (60) has rounded edges (90).

**17.** A sterilisation device according to any preceding claim comprising anti-ejection means (100)

**18.** A sterilisation device according to claim 17 wherein the anti-ejection means comprises at least one incision (100) in the thread (55).

**19.** A sterilisation device according to claim 18 having four incisions (100) two each in the two rotations of the thread (55) newest to a head (65) of the plug (50).

**20.** A sterilisation device according to claim 19 wherein the incisions (100) are equally spaced on diametrically opposing edges of the thread (55) so that the incisions in the first rotation of the thread are aligned with the incisions in the second rotation of the thread.

**21.** A sterilisation device according to any preceding claim wherein the plug (50) comprises a head (65) adapted to allow torque to be applied to the plug by a driver,

**22.** A sterilisation device according to claim 21 wherein the head (65) is polygonal.

**23.** A sterilisation device according to any preceding claim additionally comprising means for removal of the plug (70).

**24.** A sterilisation device according to claim 23 wherein the means for removal of the plug comprises an aperture (70) passing through the head (65) of the plug (50).

**25.** A sterilisation assembly comprising a sterilisation device (50) according to any preceding claim and a driver (105) configured to co-operate with the head (65) of the plug (50) facilitating the transfer of torque from the driver to the sterilisation device.

**26.** A sterilisation assembly according to claim 25 further comprising a sleeve (125).

Figure 1

EP 1 800 633 A1

Figure 2

Figure 3

95

80

90

70

65

85

55

60

figure 4

figure 5

Figure 6

105

115  120

50

← A

120

View from "A"

## Figure 7

```
┌─────────────────────────┐
│    Prepare Specimen     │
└─────────────────────────┘
             │
             ▼
┌─────────────────────────┐
│  Mount Specimen in Test │
│          Mount          │
└─────────────────────────┘
             │
             ▼
┌─────────────────────────┐
│ Apply Screw to Specimen │
│     using Applicator    │
└─────────────────────────┘
             │
             ▼
┌─────────────────────────┐
│    Place Mount in Rig   │
└─────────────────────────┘
             │
             ▼
┌─────────────────────────┐
│    Connect up Spring    │
│        Apparatus        │
└─────────────────────────┘
             │
             ▼
┌─────────────────────────┐
│      Commence Test      │
└─────────────────────────┘
```

Figure 8

Figure 9

**European Patent Office**

## PARTIAL EUROPEAN SEARCH REPORT

which under Rule 45 of the European Patent Convention shall be considered, for the purposes of subsequent proceedings, as the European search report

Application Number

EP 05 25 7920

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2004/098469 A (FEMCARE LIMITED; MAGOS, ADAM, LASZLO; FILSHIE, MARCUS) 18 November 2004 (2004-11-18)<br><br>* page 11; figures 6-10 *<br>----- | 1,2,8, 10,11, 15,17, 21,23,25 | A61F6/22<br>A61B17/12 |
| D,A | WO 97/12569 A (MAGOS, ADAM, LASZLO) 10 April 1997 (1997-04-10)<br>* the whole document *<br>----- | 1 | |
| A | GB 2 211 095 A (ANTHONY D * HAERI) 28 June 1989 (1989-06-28)<br>* the whole document *<br>----- | 1 | |
| A | EP 0 891 757 A (ETHICON, INC; GYNECARE, INC) 20 January 1999 (1999-01-20)<br>* column 7, line 22 - column 13, line 5; figures 1,6,11 *<br>----- | 1 | |

TECHNICAL FIELDS SEARCHED (IPC)

A61F
A61B

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| Munich | 1 March 2006 | Lickel, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                                            
& : member of the same patent family, corresponding document

EPO FORM 1503 03.82 (P04C07)

European Patent
Office

**INCOMPLETE SEARCH
SHEET C**

Application Number

EP 05 25 7920

Claim(s) not searched:
       27-30

Reason for the limitation of the search (non-patentable invention(s)):

Article 52 (4) EPC - Method for treatment of the human or animal body by surgery

## ANNEX TO THE EUROPEAN SEARCH REPORT
## ON EUROPEAN PATENT APPLICATION NO.

EP 05 25 7920

This annex lists the patent family members relating to the patent documents cited in the above-mentioned European search report.
The members are as contained in the European Patent Office EDP file on
The European Patent Office is in no way liable for these particulars which are merely given for the purpose of information.

01-03-2006

| Patent document cited in search report | | Publication date | Patent family member(s) | | Publication date |
|---|---|---|---|---|---|
| WO 2004098469 | A | 18-11-2004 | AU | 2004237134 A1 | 18-11-2004 |
| | | | EP | 1624836 A1 | 15-02-2006 |
| WO 9712569 | A | 10-04-1997 | AU | 7137196 A | 28-04-1997 |
| | | | GB | 2321016 A | 15-07-1998 |
| GB 2211095 | A | 28-06-1989 | NONE | | |
| EP 0891757 | A | 20-01-1999 | CA | 2243206 A1 | 18-01-1999 |
| | | | DE | 69826371 D1 | 28-10-2004 |
| | | | DE | 69826371 T2 | 29-09-2005 |
| | | | JP | 11123204 A | 11-05-1999 |
| | | | US | 5935137 A | 10-08-1999 |

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9712569 A, Magos